# EUROPEAN PATENT APPLICATION

(11) **EP 4 481 383 A1**
(43) Date of publication of application: **25.12.2024**
(21) Application number: 23775113.6
(22) Date of filing: 24.03.2023
(51) Int. Cl.: G01N 33/53, G01N 33/483

(54) **IMMUNOLOGICAL ANALYSIS METHOD, COMPLEX, METHOD FOR PRODUCING COMPLEX, AND REAGENT FOR IMMUNOLOGICAL ANALYSIS**

(30) Priority: 25.03.2022 JP 2022050800
(71) Applicant: Sekisui Medical Co., Ltd., Tokyo 103-0027 (JP)
(72) Inventor: SUNAMURA, Ei-ichiro, Tokyo 103-0027 (JP); MATSUHIRO, Shino, Tokyo 103-0027 (JP); UEMOTO, Toshiaki, Tokyo 103-0027 (JP)
(74) Representative: Maiwald GmbH
(86) International application number: PCT/JP2023/012019
(87) International publication number: WO 2023/182520

(57) **Abstract**

An immunological analysis method, including a process of binding an antibody to an antigen, the antibody being included in a complex, and the complex including a labeling substance and having a configuration in which plural antibodies are bound to each other via a scaffold compound.

## Description

### Technical Field

The present disclosure relates to an immunological analysis method, a complex, a method for producing a complex, and a reagent for immunological analysis.

### Background Art

As an immunological analysis method for biological specimens, there are various methods in which an antibody, which is bound to a labeling substance such as a luminescent substance, is allowed to bind to an antigen that is included in a biological specimen, and the concentration of the antigen is measured based on the detection result of a signal emitted from the labeling substance.

For example, Patent Document 1 describes an immunological analysis method that detects a signal emitted from a labeling substance that is bound to an antibody by means of a biotin-avidin reaction.

### Prior Art Document

### Patent Document

Patent Document 1: Japanese Patent Application Laid-Open (JP-A) No. 2010-107207

### Summary of the Invention

### Problem to be Solved by the Invention

In an immunological analysis method employing an antigen-antibody reaction, affinity of the antibody with respect to the antigen is one of the factors that affect the detection sensitivity. When the antibody fails to exhibit a sufficient degree of affinity with respect to the antigen, the antibody is likely to dissociate from the antigen to which the antibody has been bound, and a sufficient degree of detection sensitivity may not be achieved.

Further, when the immunological analysis is performed by combining plural kinds of antibodies with respect to an antigen, the location of the antigen-recognition site may affect whether the measurement is possible or the detection sensitivity.

In light of the foregoing, the present disclosure aims to provide an immunological analysis method, a complex, a method for producing a complex, and a reagent for immunological analysis, by which a favorable detection sensitivity is achieved without using an antibody that has a sufficient degree of affinity with respect to an antigen.

### Means for Solving the Problem

The means for solving the foregoing problem includes the following embodiments.
<1> An immunological analysis method, including a process of binding an antibody to an antigen, the antibody being included in a complex, and the complex including a labeling substance and having a configuration in which plural antibodies are bound to each other via a scaffold compound.
<2> The immunological analysis method according to <1>, wherein the scaffold compound is a biotin-binding protein.
<3> The immunological analysis method according to <2>, wherein the binding via the scaffold compound is a binding via biotin.
<4> The immunological analysis method according to any one of <1> to <3>, wherein the antibody has a dissociation constant with respect to the antigen of 1.00 × 10⁻⁸ or more.
<5> The immunological analysis method according to any one of <1> to <4>, wherein the process of binding the antibody to the antigen includes a process of binding a first antibody to an antigen, and a process of binding a second antibody to the antigen to which the fist antibody has been bound, and wherein the second antibody is included in the complex.
<6> The immunological analysis method according to any one of <1> to <5>, wherein the immunological analysis method is electrochemiluminescence immunoassay.
<7> A complex, including a labeling substance and having a configuration in which plural antibodies are bound to each other via a scaffold compound.
<8> A method for producing the complex according to <7>, including:
   a process of mixing an antibody to which a compound capable of binding to a scaffold compound is bound, with the scaffold compound to which a labeling substance is bound; or
   a process of mixing an antibody to which a compound capable of binding to a scaffold compound and a labeling substance are bound, with the scaffold compound.
<9> A reagent for immunological analysis, comprising the complex according to <7>.

### Effect of the Invention

According to the present disclosure, an immunological analysis method, a complex, a method for producing a complex, and a reagent for immunological analysis, by which a favorable detection sensitivity is achieved without using an antibody that has a sufficient degree of affinity with respect to an antigen, are provided.

### Brief Description of the Drawings

FIG. 1A is a graph showing results of gel filtration HPLC conducted in the Examples.
FIG. 1B is a graph showing results of gel filtration HPLC conducted in the Examples.
FIG. 2A is a graph showing results of electrochemiluminescence immunoassay using second antibody A (antigen concentration: 0 ng/mL to 2000 ng/mL).
FIG. 2B is a graph showing results of electrochemiluminescence immunoassay using second antibody A (antigen concentration: 0 ng/mL to 100 ng/mL).
FIG. 3A is a graph showing results of electrochemiluminescence immunoassay using second antibody B (antigen concentration: 0 ng/mL to 2000 ng/mL).
FIG. 3B is a graph showing results of electrochemiluminescence immunoassay using second antibody B (antigen concentration: 0 ng/mL to 100 ng/mL).
FIG. 4A is a graph showing results of electrochemiluminescence immunoassay using second antibody C (antigen concentration: 0 ng/mL to 2000 ng/mL).
FIG. 4B is a graph showing results of electrochemiluminescence immunoassay using second antibody C (antigen concentration: 0 ng/mL to 100 ng/mL).

### Embodiments for Implementing the Invention

In the present disclosure, any numerical range described using the expression "from ... to ..." represents a range in which numerical values described before and after the "to" are included in the range as a minimum value and a maximum value, respectively.

In a numerical range described in a stepwise manner, in the present disclosure, an upper limit value or a lower limit value described in one numerical range may be replaced with an upper limit value or a lower limit value in another numerical range described in a stepwise manner. Further, in a numerical range described in the present disclosure, the upper limit value or the lower limit value in the numerical range may be replaced with a value shown in the Examples.

In the present disclosure, in a case in which a respective component in a composition includes plural kinds of substances, the content of the respective component refers to the total content of the plural kinds of substances present in the composition, unless otherwise specified.

### <Immunological Analysis Method>

The immunological analysis method according to the present disclosure includes a process of binding an antibody to an antigen, and the antibody is included in a complex that includes a labeling substance and has a configuration in which plural antibodies are bound to each other via a scaffold compound.

The studies conducted by the present inventors have proved that when an antibody to be bound to an antigen is included in a complex as described above, a favorable detection sensitivity is achieved without using an antibody that has a high degree of affinity with respect to an antigen. The reason for this is thought to be as follows, for example.

When an antibody, which is bound to an antigen, is included in a complex, a further antibody exists in the vicinity of the antibody in the same complex. Therefore, if the antibody dissociates from an antigen, the likelihood that the antibody or a different antibody in the vicinity of the antibody will rebind to the antigen is higher than a case in which a conventional antibody is bound individually to an antigen. It is thought that the high likelihood of rebinding compensates for the insufficient affinity of the antibody with respect to an antigen, and contributes to an improvement in detection sensitivity.

According to the method of the present disclosure, in addition to achieving a favorable detection sensitivity without using an antibody with a high degree of affinity, it is possible to try a greater number of combinations of antibodies when studying an immunological analysis method. Therefore, for example, it is possible to efficiently promote the development of reagents for immunological analysis.

The method for conducting the immunological analysis method according to the present disclosure is not particularly limited, as long as the method detects a signal emitted from a labeling substance by way of antigen-antibody reaction.

Specific examples of the method include electrochemiluminescence immunoassay in which an electrochemiluminescent material is used as a labeling substance (ECLIA), enzyme immunoassay in which an enzyme is used as a labeling substance (EIA), radioimmunoassay in which a radioisotope is used as a labeling substance (RIA), chemiluminescence immunoassay in which a chemiluminescent material is used as a labeling substance (CIA), fluorescence immunoassay in which a fluorescent material is used as a labeling substance (FIA), and bioluminescence immunoassay in which a bioluminescent material is used as a labeling substance (BLIA).

Among these methods, the immunological analysis method according to the present disclosure is preferably conducted by electrochemiluminescence immunoassay (ECLIA).

The composite used in the immunological analysis method according to the present disclosure includes a configuration in which plural antibodies are bound to each other via a scaffold compound. The configuration can be prepared by, for example, using a compound having plural sites capable of binding to an antibody directly or indirectly, as a scaffold compound, and allowing plural antibodies to bind to the plural sites of the scaffold compound.

The scaffold compound may be a biological compound or a synthetic compound.

In an embodiment, the scaffold compound may be a biotin-binding protein. The biotin-binding protein has four sites at which biotin is bound in a molecule. Therefore, by using a biotin-binding protein as the scaffold compound, and using an antibody to which biotin is bound as the antibody, it is possible to form a complex in which plural antibodies are crosslinked via a biotin-binding protein.

Examples of the biotin-binding protein include streptavidin and avidin, and streptavidin with less non-specific binding is preferred.

The method of binding an antibody to a scaffold compound is not particularly limited, and examples thereof include a biotin-avidin method, physical adsorption, a method using hydrogen bonding, a method using van der Waals binding, and a method using a crosslinker. Among these, a method that does not require cumbersome processes for the binding is preferred.

The complex includes a labeling substance. The manner of including a labeling substance in the complex is not particularly limited. For example, a labeling substance may be bound to either one of a scaffold compound or an antibody, or may be bound to each of a scaffold compound and an antibody.

The labeling substance may be directly bound to one or both the scaffold compound and the antibody, or may be bound to one or both the scaffold compound and the antibody via an organic molecular chain or the like.

In the present disclosure, the labeling substance refers to a substance that engages in the emission of a signal for detection. The labeling substance may be a substance that emits a signal spontaneously, or may be a substance that emits a signal in an indirect manner.

The type of the labeling substance is not particularly limited, and examples thereof include known labeling substances such as electrochemiluminescent materials, enzymes, radioisotopes, chemiluminescent materials, fluorescent materials and bioluminescent materials.

When an electrochemiluminescent material is used as the labeling substance, specific examples thereof include metal complexes such as ruthenium complexes and osmium complexes.

From the viewpoint of ease of forming a complex, the molecular weight of the labeling substance is preferably less than the molecular weight of an antibody to which the labeling substance is bound. Specifically, the molecular weight of the labeling substance is preferably 150,000 or less, more preferably 10,000 or less, further preferably 5,000 or less, particularly preferably 2,000 or less.

The labeling material preferably has a size that does not sterically hinder the formation of a complex together with the plural antibodies.

In the method of the present disclosure, the process of binding an antibody to an antigen may include a process of binding a first antibody to an antigen, and a process of binding a second antibody to the antigen to which the fist antibody has been bound, wherein the second antibody is included in the complex. In other words, the method of the present disclosure may be a sandwich method.

From the viewpoint of detection sensitivity, the first antibody is preferably bound to an insoluble carrier. When the first antibody is bound to an insoluble carrier, it is possible to immobilize the complex to the insoluble carrier via the first antibody and the antigen, and detect a signal emitted from a labeling substance included in the complex in an efficient manner.

The method for detecting a signal emitted from a labeling substance may be selected depending on the type of the labeling substance.

For example, when an electrochemiluminescent material is used as the labeling substance, the labeling substance is caused to emit light via electrochemical reaction upon application of a voltage thereto, and the intensity of emission is detected.

The insoluble carrier to which the first antibody is bound may be selected depending on the method of performing the immunological analysis, and examples thereof include particles, an immunoplate and a membrane.

From the viewpoint of operability, the insoluble carrier is preferably particles, more preferably particles having a magnetic property (hereinafter, also referred to as magnetic particles).

When the insoluble carrier is in the form of particles, the average particle diameter thereof may be selected from a range of from 0.5 µm to 10 µm, preferably from 1 µm to 5 µm, more preferably from 2.5 µm to 4 µm. The foregoing average particle diameter refers to an arithmetic average value of the measured maximum diameters of 50 particles that are randomly selected from those observed with a scanning electron microscope or the like.

When the insoluble carrier is in the form of magnetic particles, the particles may be totally composed of a magnetic body, or the particles may be partly composed of a magnetic body. Examples of the particles that are partly composed of a magnetic body include those having core particles that are covered with a magnetic layer including a magnetic body.

From the viewpoint of reducing the specific weight of the magnetic particles and increasing the dispersibility thereof in a measurement sample, the magnetic particles preferably have core particles including a resin. The type of the resin is not particularly limited, and may be selected from known resins.

The magnetic body included in the magnetic layer may be selected from metals and metal oxides having magnetic properties, such as metals in periods 4-6 and groups 8-10 in the periodic table, lanthanoids and magnetic iron oxides, without particular restriction.

The method for binding the first antibody to the magnetic particles is not particularly limited. For example, the first antibody may be bound to the magnetic particles via a functional group disposed at a surface of the magnetic particles, such as an epoxy group, an amino group, a mercapto group, a carboxy group, or an acid anhydride group. The functional group may be disposed at a surface of the magnetic particles using a silane compound or the like.

As necessary, the method according to the present disclosure may include a process other than the process of binding the antibody to the antigen in a biological sample.

For example, the method may include a process of removing the antigen or the antibody that is free in a biological sample.

The method according to the present disclosure may be applied to the test of a sample obtained from a living body. Specifically, the method may be applied to the detection of a target substance (antigen), or the measurement of the concentration thereof, included in a sample.

The type of the sample obtained from a living body is not particularly limited, and may be selected from blood, blood serum, blood plasma, lymph fluid, urine, stercus, ascites fluid, pleural effusion, and cerebral spinal fluid.

The target substance included in a sample is not particularly limited, as long as it serves as the antigen.

The method according to the present disclosure can achieve a favorable detection sensitivity without using an antibody having a high affinity with respect to an antigen. For example, the method according to the present disclosure can achieve a favorable detection sensitivity even in a case in which the dissociation constant of the antibody with respect to the antigen is 1.00 × 10⁻⁸ or more.

The dissociation constant of an antibody with respect to an antigen refers to a value obtained by dividing a dissociation rate constant (Kd) by an association rate constant (Ka). The smaller the dissociation constant is, the higher the affinity of the antibody with respect to the antigen is. The dissociation constant of an antibody with respect to an antigen is measured by the method described in the Examples.

In the method according to the present disclosure, the time of the formation of a complex is not particularly limited, as long as it is prior to the process of binding an antibody to an antigen.

For example, the time of the formation of a complex may be temporally connected to the process of binding an antibody to an antigen, or may not be temporally connected to the process of binding an antibody to an antigen.

Examples of the cases in which the time of the formation of a complex is not temporally connected to the process of binding an antibody to an antigen includes a case in which the antibody is included in the complex at the stage of producing the reagent.

### <Complex and Method for Producing Complex>

The complex according to the present disclosure is a complex, including a labeling substance and having a configuration in which plural antibodies are bound to each other via a scaffold compound.

When the complex according to the present disclosure is used as the antibody for the immunological analysis method, it is possible to achieve a favorable detection sensitivity with respect to the antigen, without using an antibody having a high affinity with respect to the antigen.

The details and preferred embodiments of the complex according to the present disclosure are the same as the details and preferred embodiments of the complex used in the foregoing method according to the present disclosure.

The method for producing a complex according to the present disclosure includes:
a process of mixing an antibody to which a compound capable of binding to a scaffold compound is bound, with the scaffold compound to which a labeling substance is bound; or
a process of mixing an antibody to which a compound capable of binding to a scaffold compound and a labeling substance are bound, with the scaffold compound.

In the foregoing method, the compound capable of binding to a scaffold compound may be biotin, and the scaffold compound may be a biotin-binding protein.

Namely, the method may include:
a process of mixing an antibody to which biotin is bound with a biotin-binding protein to which a labeling substance is bound; or
a process of mixing an antibody to which a labeling substance and biotin are bound with a biotin-binding protein.

The complex in which the labeling substance is bound to the scaffold compound may be obtained by, for example, mixing the antibody to which biotin is bound with a biotin-binding protein, as the scaffold compound, to which a labeling substance is bound, and causing biotin to bind to the biotin-binding protein.

The complex in which the labeling substance is bound to the antibody may be obtained by, for example, mixing the antibody to which a labeling substance and biotin are bound with a biotin-binding protein, as the scaffold compound, and causing biotin to bind to the biotin-binding protein.

The binding of biotin or a labeling substance to the antibody or the biotin-binding protein may be performed by, for example, causing a functional group that is bound to biotin or the labeling substance, such as an NHS ester group or a sulfo-NHS ester group, to react with a primary amino group of the antibody or the biotin-binding protein.

### <Reagent for Immunological Analysis>

The reagent for immunological analysis according to the present disclosure includes the foregoing complex according to the present disclosure.

When the reagent according to the present disclosure is used as a reagent for immunological analysis, a favorable detection sensitivity can be achieved without using an antibody having high affinity with respect to an antigen.

The details and preferred embodiments of the complex included in the reagent according to the present disclosure are the same as the details and preferred embodiments of the complex used in the foregoing method according to the present disclosure.

The reagent according to the present disclosure may be a combination of a reagent including the complex and a reagent including other components (i.e., a reagent kit). Examples of the reagent including other components include a reagent including a calibrator, a standardized antigen, antibody-binding beads or the like, a sample diluent, a solution for reaction, a BF washing solution, and a luminescent electrolytic solution.

The reagent according to the present disclosure may be used in the foregoing immunological analysis method according to the present disclosure.

### Examples

In the following, the present disclosure is explained by referring to the Examples. However, the present disclosure is not restricted to the Examples.

### <Example 1>

### (Preparation of Antibody)

A mouse-derived anti-AFP monoclonal antibody, reactive to alpha-fetoprotein (AFP) as an antigen with or without a sugar chain (hereinafter, referred to as the first antibody) was prepared as the antibody to be bound to magnetic particles.

Three types of rat-derived anti-AFP monoclonal antibodies (hereinafter, referred to as the second antibodies A, B and C), specifically reactive to AFP that had been subjected to glycan cleavage using Endoglycosidase F3 (Endo F3, EC3.2.1.96) as an enzyme, were prepared as the antibodies to bind to the AFP bound to the first antibody.

The dissociation constant (KD) of the antibodies were measured using BIACORE T200 and BIACORE T200 Evaluation software (Cytiva) as an index of the affinity of the antibody with respect to the antigen. The measurement was conducted using HBS-EP buffer solution (Cytiva) at a rate of 30 µL/min. The dissociation rate constant and the association rate constant were calculated by kinetics assay of 1: 1 binding model, and the KD (M) was obtained by dividing the dissociation rate constant by the association rate constant. The results are shown in Table 1.

**Table 1**

| Antibody Type | Second Antibody A | Second Antibody B | Second Antibody C | First Antibody |
|---|---|---|---|---|
| KD (M) | 1.63×10⁻⁸ | 3.66×10⁻⁸ | 2.57×10⁻⁹ | 1.69×10⁻¹⁰ |

### (Binding of First Antibody to Magnetic Particles)

Magnetic particles were obtained from a dispersion (30 mg) of magnetic particles (Sekisui Medical, average particle diameter: 3 µm, having core particles made of resin covered with a magnetic layer, and having epoxy groups at a surface) by magnetically gathering the magnetic particles with a magnet and removing a solvent. Subsequently, the magnetic particles were washed by adding 1 mL of BPS-1 (140 mM K₂HPO₄, 10 mM KH₂PO₄, 150 mM NaCl, pH 7.8) to the magnetic particles and stirring. The magnetic particles were magnetically gathered using a magnet and a solvent was removed. The magnetic particles were mixed with 1 mL of the first antibody, at a concentration adjusted to 1 mg/mL with PBS-1, and the mixture was stirred with a rotor at 25°C for 72 hours. Thereafter, the magnetic particles were washed three times with PBS-1.

The magnetic particles were mixed with 1 mL of diluent A (50 mM HEPES, 150 mM NaCl, 10 mM EDTA3Na, 20% N102 (NOF CORPORATION), 0.1% BSA, 200 µg/mL mouse-derived IgG, 0.01% Tween 20, 0.05% ProClin 300, pH 7.2), and the mixture was stirred with a rotor at 25°C for 24 hours. The magnetic particles were washed three times with the same diluent, and 1 mL of the same diluent was added, thereby obtaining an undiluted solution containing the magnetic particles to which the first antibody was bound. The undiluted solution was diluted with diluent A to the final concentration of 1.5 mg/mL, thereby obtaining a suspension containing the magnetic particles to which the first antibody was bound.

### (Binding of Biotin to Second Antibody)

The concentration of the second antibody was adjusted to 2 mg/mL (13.3 µM, diluted with PBS-1), and the second antibody was allowed to react with EZ-Link Sulfo-NHS-LC-LC-Biotin (Thermo Fisher Scientific) by an amount 20 times as much as the second antibody (267 µM). The reaction was conducted at 25°C for 30 minutes.

The reactant was dialyzed with PBS (pH 7.2) to remove unreacted EZ-Link Sulfo-NHS-LC-Biotin, thereby obtaining a solution containing the second antibody to which biotin was bound. The dialysis was conducted twice at 4°C by an amount of 100 times as much as the reactant.

After the dialysis, the concentration of the second antibody was determined based on the absorbance measured with a spectrophotometer. The number of biotin bound to a single molecule of the antibody was determined with Pierce Biotin Quantitation Kit (Thermo Fisher Scientific). The results are shown in Table 2.

**Table 2**

| Antibody Type | Second Antibody A | Second Antibody B | Second Antibody C |
|---|---|---|---|
| Number of Biotin /molecule | 10.0 | 11.1 | 8.1 |

### (Preparation of Ruthenium Complex-Bound Streptavidin)

As a labeling substance, bis(2,2'-bipyridine)[4'-methyl-4-(4-NHS-4-oxobutyl)-2,2'-bipyridine]ruthenium (II) bis(hexafluorophosphate) (hereinafter, referred to as ruthenium complex NHS, Tokyo Chemical Industry) was used.

A streptavidin recombinant (Fujifilm Wako Pure Chemical) was subjected to solution displacement with PBS-1. The ruthenium complex NHS was added to the streptavidin recombinant that had been subjected to the solution displacement, at a molar ratio of 1:5, and the mixture was allowed to stand to react at 25°C for 30 minutes. The reaction was terminated by adding a 2M glycine solution by an amount of 6% (w/w) and allowing to stand at 25°C for 30 minutes. The reactant was dialyzed with PBS (pH 7.2) to remove the ruthenium complex-bound glycine, thereby obtaining a solution containing streptavidin to which the ruthenium complex was bound. The dialysis was conducted twice at 4°C by an amount of 100 times as much as the reactant.

The concentration of the ruthenium-labeled streptavidin was measured by BCA assay (Micro BCA protein assay, Thermo Fisher Scientific). Further, the absorbance at 455 nm was measured, and the number of the ruthenium complex bound to a single molecule of streptavidin was determined at a coefficient of molar absorbance of ruthenium = 1370. The number of the ruthenium complex bound to a single molecule of streptavidin was 3.6.

### (Preparation of Complex)

The ruthenium complex-bound streptavidin was added to diluent A at a final concentration of 25 µg/mL, and the mixture was stirred uniformly at room temperature for 5 minutes. Subsequently, the biotin-bound second antibody was added to the mixture at a final concentration of 5 µg/mL, and the mixture was stirred at room temperature for 5 minutes, thereby preparing a complex.

### (Gel Filtration HPLC Analysis)

Whether or not a complex was formed by the mixing of the ruthenium complex-bound streptavidin with the biotin-bound second antibody was confirmed by performing gel filtration HPLC analysis.

The analysis was conducted using HPLC LC-20A series (Shimadzu) connected with TSKgel G 3000 SWXI, column (Tosoh) with an eluent (25 mM phosphate buffer, 150 mM NaCl, pH 7.2) at a rate of 1 mL/min and an absorbance of 280 nm.

The analysis was directed to the biotin-bound second antibody A, the ruthenium complex-bound streptavidin, and a mixture thereof.

In order to increase the amount of detection, the mixture was prepared such that the final concentration of the ruthenium complex-bound streptavidin was 1000 µg/mL and the final concentration of the biotin-bound second antibody A was 200 µg/mL, and the mixture was diluted appropriately with an eluent.

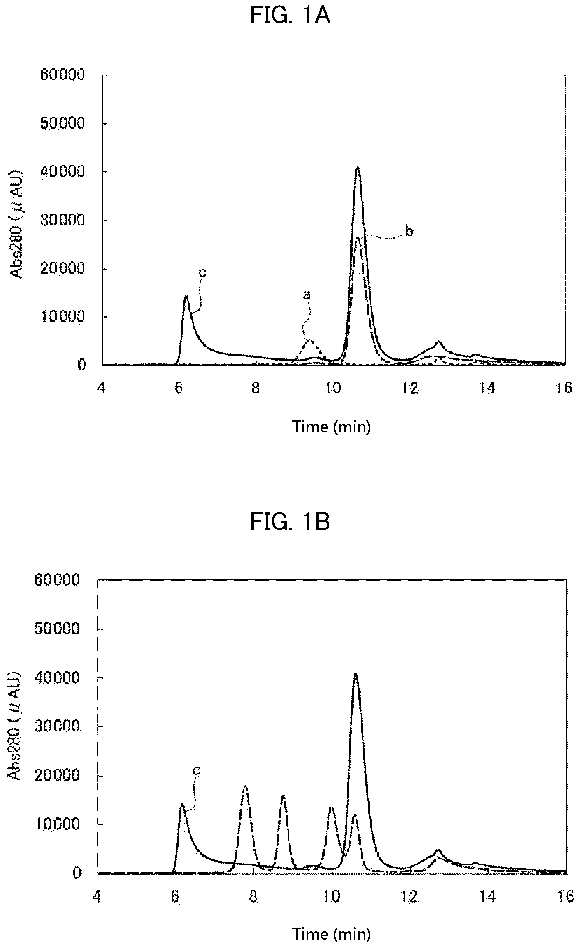

The molecular weight was determined using a gel filtration markers kit (Oriental Yeast Co., Ltd., molecular weight: 12,400-290,000). The results are shown in FIG. 1A and FIG. 1B.

In FIG. 1A, the biotin-bound second antibody A is indicated by a, the ruthenium complex-bound streptavidin is indicated by b, and the mixture thereof is indicated by c.

The dotted line in FIG. 1B refers to the peaks of the molecular weight markers. The molecular weights of the markers are 12,400, 32,000, 67,000, 142,000 and 290,000 in the order from the right.

As shown in FIG. 1A and FIG. 1B, there was a peak corresponding to the complex in which the second antibodies A (molecular weight: approximately 150,000) were bound to each other via streptavidin (molecular weight: approximately 60,000), i.e., a fraction not separable with the column used in the analysis with a molecular weight of over 500,000, confirming that the complex was included in the mixture.

### (Preparation of Measurement Sample)

A sample was prepared by diluting an AFP antigen derived from human hepatoma cell line HepG2 (Meridian) with an antigen diluent (50 mM HEPES, 150 mM NaCl, 10 mM EDTA3Na, 1.0% BSA, 0.01% Tween 20, 0.05% ProClin 300, pH 7.2).

The concentration of the AFP was determined using µTASWako AFP-L3 (Fujifilm Wako Chemicals). Specifically, the concentration of the AFP was adjusted at 2000 ng/mL based on the measurement values obtained by µTASWako AFP-L3, and the concentrations of the antigen in the samples used for the measurements were adjusted by diluting with the antigen diluent in a stepwise manner. The antigen diluent was used as a blank sample.

### (Electrochemiluminescence immunoassay)

The electrochemiluminescence immunoassay was conducted using a full-automatic electrochemiluminescence immunoassay machine Picolumi III (Sekisui Medical).

First, a measurement specimen (10 µL) was dispensed into a prescribed reaction tube, and a specimen treatment solution (10mM MES, 0.1% BSA, 20 µg/mL Endo F3 (GenScript), 0.05% ProClin 300, pH 5.5) was added thereto, and the reaction tube was allowed to stand at 37°C for 15 minutes (glycan-cleavage treatment).

A container containing the suspension of the magnetic particles to which the first antibody was bound, and a container containing a solution of the complex, were set at the predetermined positon of the machine, respectively. The measurement specimen after the completion of the glycan-cleavage treatment was set at the predetermined position of the machine, and the measurement was conducted.

During the measurement, the suspension of magnetic particles (25 µL) is dispensed into the reaction tube and allowed to cause antigen-antibody reaction at 28°C for approximately 4.8 minutes (first reaction). Subsequently, the reactant is subjected to BF separation with a prescribed BF washing solution to remove the unreacted specimen. Subsequently, the solution of the complex (100 µL) is added and allowed to cause antigen-antibody reaction at 28°C for approximately 3 minutes (second reaction: formation of sandwich). Thereafter, the reactant is subjected to BF separation with a prescribed BF washing solution to remove the unreacted second antibody.

The magnetic particles after the reaction were suspended in a solution of a prescribed substrate (tripropylamine), and allowed to cause electrochemical luminescence at a prescribed position of the machine.

### <Comparative Example 1>

The electrochemiluminescence immunoassay was conducted in the same manner as Example 1, except that the second antibody to which the ruthenium complex was directly bound was used instead of the complex. The concentration of the second antibody was adjusted to 2 µg/mL.

### (Binding of Ruthenium Complex to Second Antibody)

The second antibody was subjected to solution displacement with PBS-1 and the concentration thereof was adjusted to 2 mg/mL. The ruthenium complex NHS was added to the second antibody at a molar ratio of 1: 10, and the mixture was allowed to stand to react at 25°C for 30 minutes. The reaction was terminated by adding a 2M glycine solution by an amount of 6% (w/w) and allowing to stand at 25°C for 30 minutes. The reactant was dialyzed with PBS (pH 7.2) to remove the ruthenium complex-bound glycine, thereby obtaining a solution containing the ruthenium complex-bound second antibody.

The concentration of the ruthenium complex-bound second antibody was measured by BCA assay (Micro BCA protein assay, Thermo Fisher Scientific). Further, the absorbance at 455 nm was measured, and the number of the ruthenium complex bound to a single molecule of the antibody was determined at a coefficient of molar absorbance of ruthenium = 1370. The results are shown in Table 3.

**Table 3**

| Antibody Type | Second Antibody A | Second Antibody B | Second Antibody C |
|---|---|---|---|
| Number of Ru complex /molecule | 6.1 | 6.4 | 4.1 |

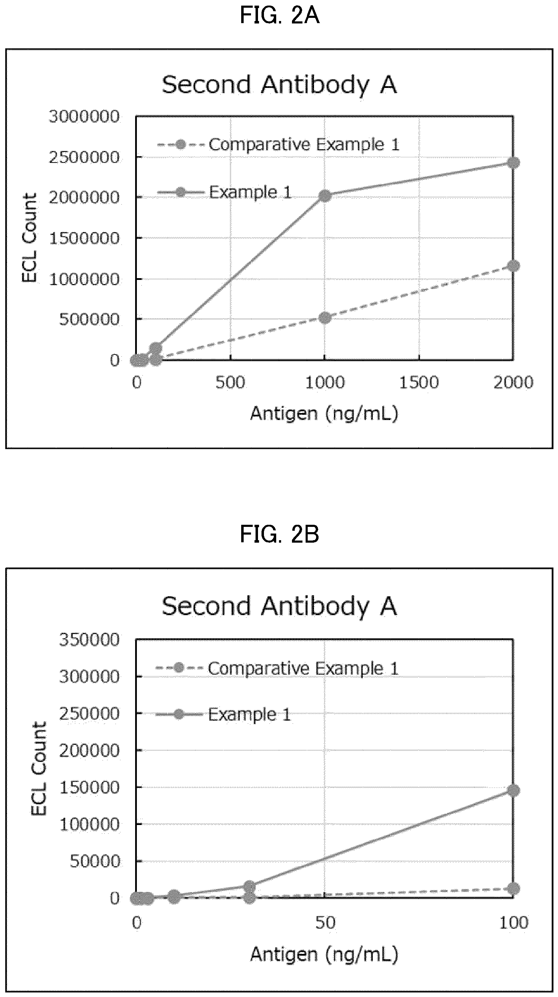

FIG. 2 to FIG. 4 are the graphs showing the results of electrochemiluminescence immunoassay conducted in Example 1 and Comparative Example 1 for comparison.

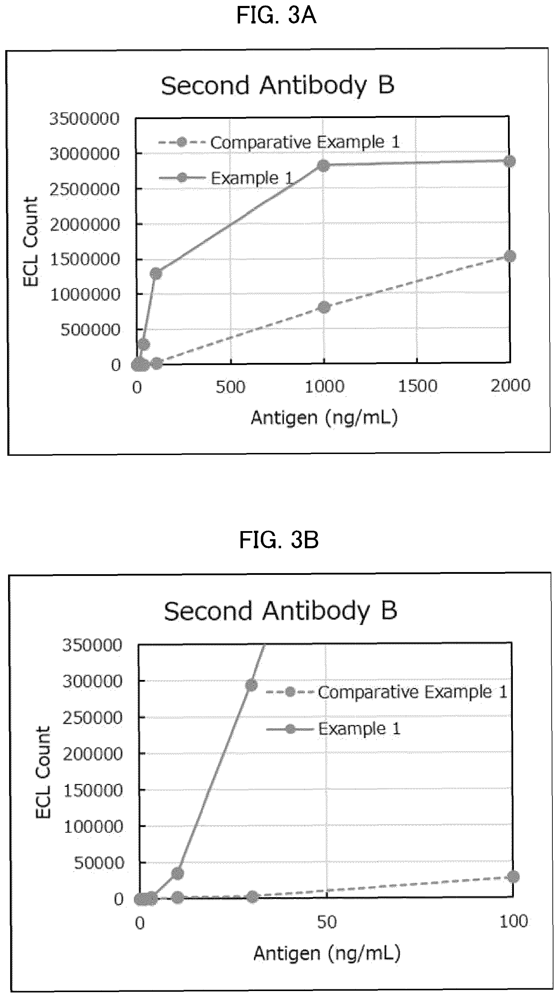

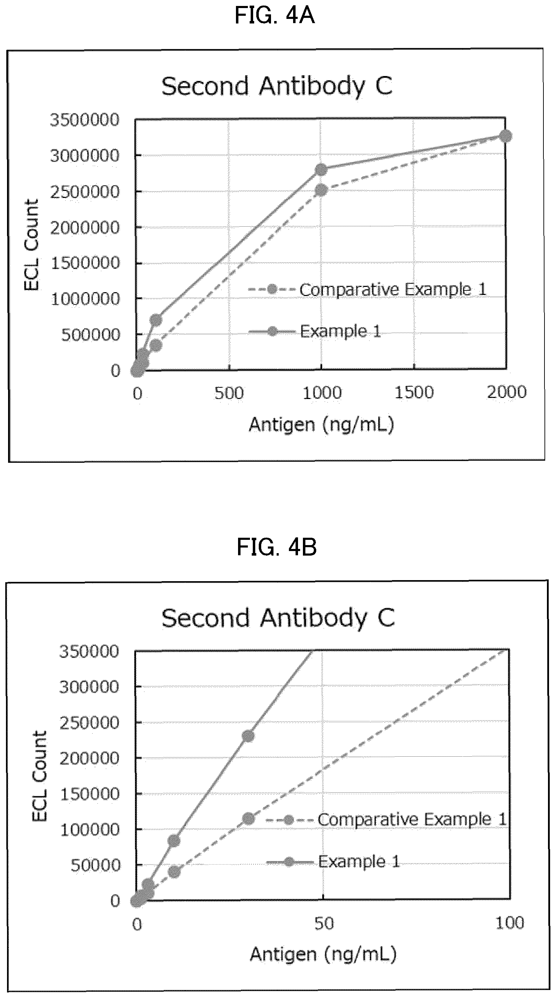

FIG. 2A and FIG. 2B are the graphs showing the results in which the second antibody A was used, FIG. 3A and FIG. 3B are the graphs showing the results in which the second antibody B was used, and FIG. 4A and FIG. 4B are the graphs showing the results in which the second antibody C was used.

In the graphs, the solid line indicates the results of Example 1 (with formation of the complex) and the dotted line indicates the results of Comparative Example 1 (without formation of the complex).

As shown in FIG. 2 to FIG. 4, Example 1, in which the analysis was conducted with the second antibody included in the complex, exhibited a higher detection sensitivity than Comparative Example 1, in which the analysis was conducted with the second antibody not included in the complex.

In cases in which the second antibody A and the second antibody B were used, Comparative Example 1 did not exhibit an antigen concentration-dependent increase in count up to 30 ng/mL, failing to conduct a precise measurement.

Example 1 exhibited an antigen concentration-dependent increase in count from 1 ng/mL, successfully conducting a precise measurement.

In cases in which the second antibody C, having a smaller dissociation constant than the second antibody A and the second antibody B, while Comparative Example 1 exhibited an antigen concentration-dependent increase in count from 1 ng/mL, the values thereof was approximately half as much as the values of Example 1.

### <Comparative Example 2>

The electrochemiluminescence immunoassay was conducted in the same manner as Example 1, except that the second antibody was allowed to react with the antigen (formation of sandwich) without the formation of the complex, and subsequently the ruthenium complex was bound to the second antibody via streptavidin.

A measurement specimen (30 µL) was dispensed into a prescribed reaction tube, and a specimen treatment solution (10mM MES, 0.1% BSA, 20 µg/mL Endo F3 (GenScript), 0.05% ProClin 300, pH 5.5) was added thereto, and the reaction tube was allowed to stand at 37°C for 15 minutes (glycan-cleavage treatment).

A container containing the suspension of the magnetic particles to which the first antibody was bound, and a container containing a solution of the ruthenium complex-bound streptavidin (20 µg/mL, diluent A was used as the solvent), were set at the predetermined position of the machine.

To the measurement specimen after the completion of the glycan-cleavage treatment, 25 µL of a solution of the biotin-bound second antibody A (20 µg/mL, diluent A was used as the solvent) was added. The mixture was set at the predetermined position of the machine, and the measurement was conducted.

During the measurement, the antigen is allowed to react with the second antibody for 1.2 minutes from the start of the measurement (first reaction). Subsequently, the suspension of the magnetic particles (25 µL) is dispensed into the reaction tube, and the antigen is allowed to react with the first antibody at 28°C for approximately 4.8 minutes (second reaction, formation of sandwich). Subsequently, the reactant is subjected to BF separation with a prescribed BF washing solution to remove the unreacted antigen and the unreacted second antibody. Subsequently, a solution of the ruthenium-labeled streptavidin (100 µL) is added to the reaction tube, and streptavidin is allowed to bind to biotin that is bound to the second antibody (third reaction). Thereafter, the reactant was subjected to BF separation with a prescribed BF washing solution to remove the unreacted ruthenium-labeled streptavidin.

The complex generated was suspended in a solution of a prescribed substrate (tripropylamine), and allowed to cause electrochemical luminescence.

The results in which the second antibody A was used as the second antibody are shown in Table 4. The count at respective antigen concentrations in Table 4 refers to a value calculated by subtracting a blank count from the count obtained by the measurement (specific count). The blank count shown in Table 4 refers to a value measured at an antigen concentration of 0 ng/mL.

**Table 4**

| Antigen (ng/mL) | Comparative Example 1 | Comparative Example 2 |
|---|---|---|
| 0.5 | 51.8 | 31.2 |
| 1 | 123.6 | 57.4 |
| 10 | 5945.3 | 906.8 |
| 100 | 259609 | 6949.9 |
| Blank Count | 269.9 | 382.2 |

As shown in Table 4, Comparative Example 2, in which a labeling substance was bound to the second antibody via streptavidin, after binding the second antibody to the antigen, exhibited a lower detection sensitivity than Comparative Example 1, in which the ruthenium complex was directly bound to the second antibody.

### <Comparative Example 3>

Samples were prepared at different concentrations of the second antibody (5 µg/mL to 40 µg/mL) by the method described in Comparative Example 1. As the second antibody, the second antibody B was used.

The electrochemiluminescence immunoassay was conducted on the samples in the same manner as Comparative Example 1. The results are shown in Table 5.

The "blank deduction" in Table 5 refers to a specific count value, which is a value obtained from subtracting a blank count from the count, and the "S/N ratio" refers to a value obtained by dividing the count by the blank count.

**Table 5**

| Antigen (ng/mL) | | Second Antibody (µg/mL) | | | |
|---|---|---|---|---|---|
| | | 5 | 10 | 20 | 40 |
| Count | 0 | 261.0 | 279.8 | 393.8 | 500.1 |
| | 1 | 396.9 | 363.0 | 469.1 | 604.4 |
| | 10 | 2852.3 | 2787.4 | 3287.9 | 3358.8 |
| | 100 | 132638.7 | 131376.0 | 141508.5 | 135465.3 |
| | 1000 | 1976947.7 | 2043923.3 | 2079213.5 | 2115883.2 |
| Specific Count (Blank Deduction) | 1 | 135.9 | 83.2 | 75.3 | 104.3 |
| | 10 | 2591.3 | 2507.6 | 2894.1 | 2858.7 |
| | 100 | 132377.7 | 131096.2 | 141114.7 | 134965.2 |
| | 1000 | 1976686.7 | 2043643.5 | 2078819.7 | 2115383.1 |
| S/N Ratio | 1 | 1.52 | 1.3 | 1.19 | 1.21 |
| | 10 | 10.93 | 9.96 | 8.35 | 6.72 |
| | 100 | 508.19 | 469.54 | 359.34 | 270.88 |
| | 1000 | 7574.51 | 7304.94 | 5279.87 | 4230.92 |

As shown in Table 5, while the values of the specific count (blank deduction) did not change significantly even with the changes in the concentration of the second antibody, the values of the blank count exhibited an increase alone. In addition, the values of the S/N ratio decreased as the concentration of the second antibody was increased, indicating the worsening of the S/N ratio.

### <Comparative Example 4>

Samples were prepared at different numbers of the ruthenium complex to bind to the second antibody by the method described in Comparative Example 1.

Specifically, the reaction molar ratio of the second antibody and the ruthenium complex NHS was set at 1:10 or 1:20. The number of the ruthenium complex with respect to a single molecule of the antibody was 6.4 or 10.2. As the second antibody, the second antibody B was used.

The electrochemiluminescence immunoassay was conducted on the samples in the same manner as Comparative Example 1, in which the concentration of the second antibody was fixed at 5 µg/mL. The results are shown in Table 6.

**Table 6**

| Antigen (ng/mL) | | Reaction Molar Ratio | |
|---|---|---|---|
| | | 1:10 | 1:20 |
| Count | 0 | 261.0 | 465.2 |
| | 1 | 396.9 | 546.5 |
| | 10 | 2852.3 | 3447.0 |
| | 100 | 132638.7 | 162805.5 |
| | 1000 | 1976947.7 | 2552327.5 |
| Specific Count (Blank Deduction) | 1 | 135.9 | 81.3 |
| | 10 | 2591.3 | 2981.8 |
| | 100 | 132377.7 | 162340.3 |
| | 1000 | 1976686.7 | 2551862.3 |
| S/N Ratio | 1 | 1.52 | 1.17 |
| | 10 | 10.93 | 7.41 |
| | 100 | 508.19 | 349.97 |
| | 1000 | 7574.51 | 5486.52 |

As shown in Table 6, the specific count (blank deduction) at a reaction molar ratio of 1:20 was lower than that at a reaction molar ratio of 1:10, at a low antigen concentration (1 ng/mL).

In addition, the value of S/N ratio at a reaction molar ratio of 1:20 was lower than that at a reaction molar ratio of 1:10, indicating the worsening of the S/N ratio.

From the results of Comparative Example 1 and Example 1, it was found that the state of the second antibody being included in the complex contributes to an improvement in the detection sensitivity in the immunological analysis method. Further, it was found that the detection sensitivity is improved even when the second antibody did not have a high affinity with respect to an antigen.

From the results of Comparative Examples 2-4, it was found that the binding a labeling substance to the second antibody to which an antigen had been bound; the increasing the concentration of the second antibody in the sample; and the increasing the number of a labeling substance to bind to the second antibody do not significantly contribute to an improvement in the detection sensitivity in the immunological analysis method. The reason for this is thought to be that the measures studied in Comparative Examples 2-4 fail to adequately compensate for an insufficiency in the affinity of the antibody with respect to an antigen.

The disclosure of Japanese Patent Application No. 2022-050800 is incorporated herein by reference in its entirety.

All publications, patent applications, and technical standards mentioned in the present specification are incorporated herein by reference to the same extent as if each individual publication, patent application, or technical standard was specifically and individually indicated to be incorporated by reference.

## Claims

1. An immunological analysis method, comprising a process of binding an antibody to an antigen, the antibody being included in a complex, and the complex comprising a labeling substance and having a configuration in which plural antibodies are bound to each other via a scaffold compound.

2. The immunological analysis method according to claim 1, wherein the scaffold compound is a biotin-binding protein.

3. The immunological analysis method according to claim 2, wherein the binding via the scaffold compound is a binding via biotin.

4. The immunological analysis method according to any one of claim 1 to claim 3, wherein the antibody has a dissociation constant with respect to the antigen of 1.00 × 10⁻⁸ or more.

5. The immunological analysis method according to claim 1, wherein the process of binding the antibody to the antigen comprises a process of binding a first antibody to an antigen, and a process of binding a second antibody to the antigen to which the fist antibody has been bound, and wherein the second antibody is included in the complex.

6. The immunological analysis method according to claim 1, wherein the immunological analysis method is electrochemiluminescence immunoassay.

7. A complex, comprising a labeling substance and having a configuration in which plural antibodies are bound to each other via a scaffold compound.

8. A method for producing the complex according to claim 7, comprising:
a process of mixing an antibody to which a compound capable of binding to a scaffold compound is bound, with the scaffold compound to which a labeling substance is bound; or
a process of mixing an antibody to which a compound capable of binding to a scaffold compound and a labeling substance are bound, with the scaffold compound.

9. A reagent for immunological analysis, comprising the complex according to claim 7.
